(19) Europäisches Patentamt
European Patent Office
Office européen des brevets

(11) EP 3 339 431 A1

(12) **EUROPEAN PATENT APPLICATION**

(43) Date of publication:
**27.06.2018 Bulletin 2018/26**

(51) Int Cl.:
***C12N 9/04*** *(2006.01)* ***C12N 15/52*** *(2006.01)*

(21) Application number: **16206479.4**

(22) Date of filing: **22.12.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Roche Diabetes Care GmbH**
  **68305 Mannheim (DE)**
  Designated Contracting States:
  **DE**
- **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO RS SE SI SK SM TR**

(72) Inventor: **The designation of the inventor has not yet been filed**

(74) Representative: **Meinel, Anne Julia et al**
**Roche Diabetes Care GmbH**
**Sandhofer Straße 116**
**68305 Mannheim (DE)**

# (54) GLUCOSE DEHYDROGENASE VARIANTS WITH IMPROVED PROPERTIES

(57) The present invention relates to improved variants of variants of the Glucose Dehydrogenases (GlucDH) derived from *Bacillus subtilis* having improved properties in the presence of cNAD as cofactor, to genes encoding such variant GlucDHs, to proteins of such GlucDH variants, and to different applications of these GlucDH variants, particularly for determining concentrations of sugars, especially of glucose, in samples such as bodily fluids, especially blood.

EP 3 339 431 A1

SEQUENCE LISTING

<110> Roche Diabetes Care GmbH
      F. Hoffmann-La Roche AG

<120> GLUCOSE DEHYDROGENASE VARIANTS WITH IMPROVED PROPERTIES

<130> P-32004-EP

<160> 5

<170> BiSSAP 1.3.6

<210> 1
<211> 261
<212> PRT
<213> Bacillus subtilis

<400> 1

```
Met Tyr Pro Asp Leu Lys Gly Lys Val Val Ala Ile Thr Gly Ala Ala
1               5                   10                  15
Ser Gly Leu Gly Lys Ala Met Ala Ile Arg Phe Gly Lys Glu Gln Ala
            20                  25                  30
Lys Val Val Ile Asn Tyr Tyr Ser Asn Lys Gln Asp Pro Asn Glu Val
        35                  40                  45
Lys Glu Glu Val Ile Lys Ala Gly Gly Glu Ala Val Val Val Gln Gly
    50                  55                  60
Asp Val Thr Lys Glu Glu Asp Val Lys Asn Ile Val Gln Thr Ala Ile
65                  70                  75                  80
Lys Glu Phe Gly Thr Leu Asp Ile Met Ile Asn Asn Ala Gly Leu Glu
                85                  90                  95
Asn Pro Val Pro Ser His Glu Met Pro Leu Lys Asp Trp Asp Lys Val
            100                 105                 110
Ile Gly Thr Asn Leu Thr Gly Ala Phe Leu Gly Ser Arg Glu Ala Ile
        115                 120                 125
Lys Tyr Phe Val Glu Asn Asp Ile Lys Gly Asn Val Ile Asn Met Ser
    130                 135                 140
Ser Val His Glu Val Ile Pro Trp Pro Leu Phe Val His Tyr Ala Ala
145                 150                 155                 160
Ser Lys Gly Gly Ile Lys Leu Met Thr Glu Thr Leu Ala Leu Glu Tyr
                165                 170                 175
Ala Pro Lys Gly Ile Arg Val Asn Asn Ile Gly Pro Gly Ala Ile Asn
            180                 185                 190
Thr Pro Ile Asn Ala Glu Lys Phe Ala Asp Pro Lys Gln Lys Ala Asp
        195                 200                 205
Val Glu Ser Met Ile Pro Met Gly Tyr Ile Gly Glu Pro Glu Glu Ile
    210                 215                 220
Ala Ala Val Ala Val Trp Leu Ala Ser Lys Glu Ser Ser Tyr Val Thr
225                 230                 235                 240
Gly Ile Thr Leu Phe Ala Asp Gly Gly Met Thr Gln Tyr Pro Ser Phe
                245                 250                 255
Gln Ala Gly Arg Gly
            260
```

<210> 2
<211> 261
<212> PRT
<213> Artificial Sequence

<220>
<223> Mutant GlucDH

<400> 2
Met Tyr Pro Asp Leu Lys Gly Lys Val Val Ala Ile Thr Gly Ala Ala
1 5 10 15
Ser Gly Leu Gly Lys Ala Met Ala Ile Arg Phe Gly Lys Glu Gln Ala
20 25 30
Lys Val Val Ile Asn Tyr Tyr Ser Asn Lys Gln Asp Pro Asn Glu Val
35 40 45
Lys Glu Glu Val Ile Lys Ala Gly Gly Glu Ala Val Val Val Gln Gly
50 55 60
Asp Val Thr Lys Glu Glu Asp Val Lys Asn Ile Val Gln Thr Ala Ile
65 70 75 80
Lys Glu Phe Gly Thr Leu Asp Ile Met Ile Asn Asn Ala Gly Leu Glu
85 90 95
Asn Pro Val Pro Ser His Glu Met Pro Leu Lys Asp Trp Asp Lys Val
100 105 110
Ile Gly Thr Asn Leu Thr Gly Ala Phe Leu Gly Ser Arg Glu Ala Ile
115 120 125
Lys Tyr Phe Val Glu Asn Asp Ile Lys Gly Asn Val Ile Asn Met Ser
130 135 140
Ser Val His Glu Val Ile Pro Trp Pro Leu Phe Val His Tyr Ala Ala
145 150 155 160
Ser Lys Gly Gly Ile Lys Leu Met Thr Lys Thr Leu Ala Leu Glu Tyr
165 170 175
Ala Pro Lys Gly Ile Arg Val Asn Asn Ile Gly Pro Gly Ala Ile Asn
180 185 190
Thr Pro Ile Asn Ala Glu Lys Phe Ala Asp Pro Lys Gln Lys Ala Asp
195 200 205
Val Glu Ser Met Ile Pro Met Gly Tyr Ile Gly Glu Pro Glu Glu Ile
210 215 220
Ala Ala Val Ala Val Trp Leu Ala Ser Lys Glu Ser Ser Tyr Val Thr
225 230 235 240
Gly Ile Thr Leu Phe Ala Asp Gly Gly Met Thr Leu Tyr Pro Ser Phe
245 250 255
Gln Ala Gly Arg Gly
260

<210> 3
<211> 261
<212> PRT
<213> Artificial Sequence

<220>
<223> Mutant GlucDH

<400> 3
Met Tyr Pro Asp Leu Lys Gly Lys Val Val Ala Ile Thr Gly Ala Ala
1 5 10 15
Ser Gly Leu Gly Lys Ala Met Ala Ile Arg Phe Gly Lys Glu Gln Ala
20 25 30
Lys Val Val Ile Asn Tyr Tyr Ser Asn Lys Gln Asp Pro Asn Glu Val
35 40 45
Lys Glu Glu Val Ile Lys Ala Gly Gly Glu Ala Val Val Val Gln Gly
50 55 60
Asp Val Thr Lys Glu Glu Asp Val Lys Asn Ile Val Gln Thr Ala Ile
65 70 75 80
Lys Glu Phe Gly Thr Leu Asp Ile Met Ile Asn Asn Ala Gly Leu Glu
85 90 95
Asn Pro Val Pro Ser His Glu Met Pro Leu Lys Asp Trp Asp Lys Val
100 105 110
Ile Gly Thr Asn Leu Thr Gly Ala Phe Leu Gly Ser Arg Glu Ala Ile
115 120 125

```
Lys Tyr Phe Val Glu Asn Asp Ile Lys Gly Asn Val Ile Asn Met Ser
    130                 135                 140
Ser Val His Glu Val Ile Pro Trp Pro Leu Phe Val His Tyr Ala Ala
145                 150                 155                 160
Ser Lys Ala Gly Ile Lys Leu Met Thr Lys Thr Leu Ala Leu Glu Tyr
                165                 170                 175
Ala Pro Lys Gly Ile Arg Val Asn Asn Ile Gly Pro Gly Ala Ile Asn
            180                 185                 190
Thr Pro Ile Asn Ala Glu Lys Phe Ala Asp Pro Lys Gln Lys Ala Asp
        195                 200                 205
Val Glu Ser Met Ile Pro Met Gly Tyr Ile Gly Glu Pro Glu Trp Ile
    210                 215                 220
Ala Ala Val Ala Val Trp Leu Ala Ser Lys Glu Ser Asn Tyr Val Thr
225                 230                 235                 240
Gly Ile Thr Leu Phe Ala Asp Gly Gly Met Thr Leu Tyr Pro Ser Phe
                245                 250                 255
Gln Ala Gly Arg Gly
            260

<210> 4
<211> 261
<212> PRT
<213> Artificial Sequence


<220>
<223> Mutant GlucDH

<400> 4
Met Tyr Pro Asp Leu Lys Gly Lys Val Val Ala Ile Thr Gly Ala Ala
1               5                   10                  15
Ser Gly Leu Gly Lys Ala Met Ala Ile Arg Phe Gly Lys Glu Gln Ala
            20                  25                  30
Lys Val Val Ile Asn Tyr Tyr Ser Asn Lys Gln Asp Pro Asn Glu Val
        35                  40                  45
Lys Glu Glu Val Ile Lys Ala Gly Gly Glu Ala Val Val Val Gln Gly
    50                  55                  60
Asp Val Thr Lys Glu Glu Asp Val Lys Asn Ile Val Gln Thr Ala Ile
65                  70                  75                  80
Lys Glu Phe Gly Thr Leu Asp Ile Met Ile Asn Asn Ala Gly Ile Glu
                85                  90                  95
Ser Pro Val Pro Ser His Glu Met Pro Leu Lys Asp Trp Asp Lys Val
            100                 105                 110
Ile Gly Thr Asn Leu Thr Gly Ala Phe Leu Gly Ser Arg Glu Ala Ile
        115                 120                 125
Lys Tyr Phe Val Glu Asn Asp Ile Lys Gly Asn Val Ile Asn Met Ser
    130                 135                 140
Ser Val His Glu Val Ile Pro Trp Pro Leu Phe Val His Tyr Ala Ala
145                 150                 155                 160
Ser Lys Gly Gly Ile Lys Leu Met Thr Lys Thr Leu Ala Leu Glu Tyr
                165                 170                 175
Ala Pro Lys Gly Ile Arg Val Asn Asn Ile Gly Pro Gly Ala Ile Asn
            180                 185                 190
Thr Pro Ile Asn Ala Glu Lys Phe Ala Asp Pro Lys Gln Lys Ala Asp
        195                 200                 205
Val Glu Ser Met Ile Pro Met Gly Tyr Ile Gly Glu Pro Glu Glu Ile
    210                 215                 220
Ala Ala Val Ala Val Trp Leu Ala Ser Lys Glu Ser Ser Tyr Val Thr
225                 230                 235                 240
Gly Ile Thr Leu Phe Ala Asp Gly Gly Met Thr Leu Tyr Pro Ser Phe
                245                 250                 255
Gln Ala Gly Arg Gly
            260
```

<210> 5
<211> 261
<212> PRT
<213> Artificial Sequence

<220>
<223> Mutant GlucDH

<400> 5
```
Met Tyr Pro Asp Leu Lys Gly Lys Val Val Ala Ile Thr Gly Ala Ala
1               5                   10                  15
Ser Gly Leu Gly Lys Ala Met Ala Ile Arg Phe Gly Lys Glu Gln Ala
            20                  25                  30
Lys Val Val Ile Asn Tyr Glu Ser Asn Lys Gln Asp Pro Asn Glu Val
        35                  40                  45
Lys Glu Glu Val Ile Lys Ala Gly Gly Glu Ala Val Val Val Gln Gly
    50                  55                  60
Asp Val Thr Lys Glu Glu Asp Val Lys Asn Ile Val Gln Thr Ala Leu
65                  70                  75                  80
Lys Glu Phe Gly Thr Leu Asp Ile Met Ile Asn Asn Ala Gly Ile Glu
                85                  90                  95
Asn Pro Val Pro Ser His Glu Met Pro Leu Lys Asp Trp Asp Lys Val
            100                 105                 110
Ile Gly Thr Asn Leu Thr Gly Ala Phe Leu Gly Ser Arg Glu Ala Ile
        115                 120                 125
Lys Tyr Phe Val Glu Asn Asp Ile Lys Gly Asn Val Ile Asn Met Ser
    130                 135                 140
Ser Val His Glu Val Ile Pro Trp Pro Leu Phe Val His Tyr Ala Ala
145                 150                 155                 160
Ser Lys Ala Gly Ile Lys Leu Met Thr Lys Thr Leu Ala Leu Glu Tyr
                165                 170                 175
Ala Pro Lys Gly Ile Arg Val Asn Asn Ile Gly Pro Gly Ala Ile Asn
            180                 185                 190
Thr Pro Ile Asn Ala Glu Lys Phe Ala Asp Pro Lys Gln Lys Ala Asp
        195                 200                 205
Val Glu Ser Met Ile Pro Met Gly Tyr Ile Gly Glu Pro Glu Trp Ile
    210                 215                 220
Ala Ala Val Ala Val Trp Leu Ala Ser Lys Glu Ser Asn Tyr Val Thr
225                 230                 235                 240
Gly Ile Thr Leu Phe Ala Asp Gly Gly Met Thr Leu Tyr Pro Ser Phe
                245                 250                 255
Gln Ala Gly Arg Gly
            260
```

**Description**

FIELD OF THE INVENTION

The present invention relates to variants of the Glucose Dehydrogenases (GlucDH) derived from *Bacillus subtilis* having improved properties in the presence of cNAD as cofactor, to genes encoding such variant GlucDHs, to proteins of such GlucDH variants, and to different applications of these GlucDH variants, particularly for determining concentrations of sugars, especially of glucose, in samples such as bodily fluids, especially blood.

BACKGROUND OF THE INVENTION

The determination of blood glucose concentration is extremely important in clinical diagnosis and in the management of diabetes. Approximately 150 million people worldwide suffer from the chronic disease *diabetes mellitus,* a figure that may double by 2025 according to the WHO. Although diabetes is readily diagnosed and treated, successful long-term management requires low-cost diagnostic tools that rapidly and accurately report blood glucose concentrations.

Diagnostic test elements are usually manufactured for use in near-patient applications. Therefore, the elements must be robust with respect to handling and storage. This applies, in particular, for the test chemistry of the test elements such as test strips or sensors / electrodes (see Hones 2008, Diabetes Technology & Therapeutics 10: S10). However, many diagnostic test elements are based on a rather complex enzyme test chemistry present on the test element. Herewith, analytes, e.g. metabolites or substrates, may be determined directly or indirectly with the aid of an enzyme. The analytes are converted with the aid of an enzyme-coenzyme complex and subsequently quantified. In this process the analyte to be determined is brought into contact with a suitable enzyme and a coenzyme where the enzyme is usually used in catalytic amounts. The coenzyme is changed, e.g., oxidized or reduced by the enzymatic reaction. This process can be detected electrochemically or photometrically either directly or by means of a mediator. A calibration provides a correlation between the measured value and the concentration of the analyte to be determined. Exemplary test elements and test chemistries are provided, e.g., in EP 0 354 441 A2, EP 0 431 456 A1, EP 0 302 287 A2, and EP 1 593 434 A2.

However, it has become apparent that such measuring systems often are characterized by a limited shelf-life and by special requirements for the environment such as cooling or dry storage in order to achieve this storage life. In order to avoid erroneous results caused by incorrect, unnoticed, faulty storage and to increase test chemistry stability particular components of the test chemistry were subjected to further developments. One approach was to increase the stability of the coenzyme and the coenzyme-enzyme complex. Coenzymes are organic molecules which are covalently or non-covalently bound to an enzyme and are changed by the conversion of the analyte. Prominent examples of coenzymes are nicotinamide adenine dinucleotide (NAD) and nicotinamide adenine dinucleotide phosphate (NADP) from which NADH and NADPH respectively are formed by reduction (N. J. Oppenheimer in The Pyridine Nucleotide Coenzymes Academic Press, New York, London 1982, J. Everese, B. Anderson, K. Yon, Editors, chapter 3, pages 56-65). It has been shown, that the coenzymes NAD and NADP are already hydrolyzed solely by the ambient humidity potentially resulting in inaccuracies when measuring analytes. As a solution to this problem stable NAD/NADH derivatives, such as carba-NAD (cNAD), have been developed and implemented in test chemistries that show improved stability and enhanced shelf-life (see, e.g., WO 2007/01249). The enzymatic synthesis of carba-NADs comprising a carbacyclic sugar instead of ribose is provided in WO 2011/012270. Further, it has been shown that stable coenzymes like carba-NAD can stabilize the Glucose Dehydrogenase enzyme when stored over a longer period of time at elevated temperatures and increased humidity (see, WO 2009/103540).

An essential component within the test chemistry is the enzyme or the enzyme system converting the analytes, e.g. metabolites or substrates, thereby enabling the direct or indirect determination and quantification of the analytes. Herein, various enzyme systems for detecting and quantitating sugars, particularly glucose, from biological samples, such as blood samples, are known in the art. One of these test formats includes the use of the enzyme Glucose Dehydrogenase with NAD as coenzyme for detecting glucose, in which case a reduced coenzyme NADH is formed. NADH may, e.g., be detected by optical methods, such as by photometric or fluorometric determination after UV excitation. An exemplary test system is described in US 2005/0214891. The purification and characterization of a suitable Glucose Dehydrogenase (GlucDH) from *Bacillus subtilis* (E.C. 1.1.1.47) expressed in Escherichia coli has been reported previously (see Hilt et al., Biochim Biophys Acta. 1991 Jan 29;1076(2):298-304). This ß-D-Glucose-NAD-1-Oxidoreduktase catalyzes the reaction of ß-D-Glucose in the presence of $NAD^+$ to Gluconolactone, NADH and $H^+$. However, the wild type GlucDH enzyme has been shown to lack thermal and hydrolytic stability under warm and humid environmental conditions. In order to improve the stability of Glucose Dehydrogenases WO 2009/103540 proposes to introduce amino acid substitutions in at least one position selected from the group of amino acid positions 96, 170 and 252, while indicating that besides these mutants the GlucDH should not comprise any further mutations. It is further disclosed that GlucDH mutants carrying a substitution at positions 96 (amino acid residue glutamic acid to amino acid residue glycine) and 170 (amino acid residue glutamic acid to amino acid residue arginine or lysine) or at positions 170 (amino acid residue glutamic acid

to amino acid residue arginine or lysine) and 252 (amino acid residue lysine to amino acid residue leucine) are preferred. The indicated mutations are also published in S.-H. Baik et al, 2003, Appl. Microbial Biotechnol., 61, 329-335.

However, when analyzing the wild type GlucDH enzyme derived from *Bacillus subtilis* and the mutant GlucDH enzymes disclosed in WO 2009/103540 for their capability to convert glucose in the presence of the artificial coenzyme cNAD it became apparent that neither of these enzymes provided the desired efficiency in enzyme activity. Hence, in order to provide a test chemistry that shows the desired thermal stability and the desired enzyme efficiency GlucDH variants exhibiting improved enzyme performance of catalytic conversion of glucose in the presence of cNAD, wherein the variant exhibits high affinity towards glucose with cNAD as coenzyme combined with high enzymatic stability were needed.

SUMMARY OF THE INVENTION

This task is solved by variants of a Glucose Dehydrogenase (GlucDH) derived from *Bacillus subtilis* provided herein. Particular embodiments, which might be realized in an isolated fashion or in any arbitrary combination, are listed in the dependent claims.

In one aspect the invention relates to a variant of a Glucose Dehydrogenase (GlucDH) derived from *Bacillus subtilis,* wherein said variant comprises an amino acid sequence having at least 90% identity to SEQ ID NO:1 and wherein said variant comprises a substitution of amino acid residue glutamic acid at a position corresponding to position 170 of SEQ ID NO: 1 with an amino acid residue lysine and a substitution of amino acid residue glutamine at a postion corresponding to position 252 of SEQ ID NO: 1 with an amino acid residue leucine, and wherein said variant further comprises one or more additional amino acid substitutions selected from the group consisting of a substitution of amino acid residue leucine at a position corresponding to position 95 of SEQ ID NO: 1 with an amino acid residue isoleucine or valine, a substitution of amino acid residue asparagine at a position corresponding to position 97 of SEQ ID NO: 1 with an amino acid residue serine, a substitution of amino acid residue glycine at a position corresponding to position 163 of SEQ ID NO: 1 with an amino acid residue alanine, a substitution of amino acid residue glutamic acid at a position corresponding to position 223 of SEQ ID NO: 1 with an amino acid residue phenylalanine, tryptophan, isoleucine, leucine, threonine or tyrosine, and a substitution of amino acid residue serine at a position corresponding to position 237 of SEQ ID NO: 1 with an amino acid residue glutamic acid, arginine or asparagine.

In one embodiment said variant comprises the additional amino acid substitutions of amino acid residue glutamic acid at the position corresponding to position 223 of SEQ ID NO: 1 with an amino acid residue phenylalanine, tryptophan, isoleucine, leucine, threonine or tyrosine, and of amino acid residue serine at the position corresponding to position 237 of SEQ ID NO: 1 with an amino acid residue glutamic acid, arginine or asparagine. In yet another embodient said variant comprises the further additional amino acid substitution of amino acid residue glycine at the position corresponding to position 163 of SEQ ID NO:1 with an amino acid residue alanine.

In another embodiment any of said recited variants further comprises one or more additional amino acid substitutions, wherein the amino acid at a position corresponding to

- position 39 of SEQ ID NO: 1 is substituted with Glu (39Glu);
- position 40 of SEQ ID NO: 1 is substituted with Cys (40Cys);
- position 46 of SEQ ID NO: 1 is substituted with Asp (46Asp);
- position 70 of SEQ ID NO: 1 is substituted with Cys (70Cys);
- position 78 of SEQ ID NO: 1 is substituted with Ala (78Ala);
- position 80 of SEQ ID NO: 1 is substituted with Leu (80Leu);
- position 96 of SEQ ID NO: 1 is substituted with Leu (96Leu),Gln (96Gln), Val (96Val), or Met (96Met);
- position 107 of SEQ ID NO: 1 is substituted with Glu (107Glu);
- position 134 of SEQ ID NO: 1 is substituted with Glu (134Glu);
- position 178 of SEQ ID NO: 1 is substituted with Ser (178Ser);
- position 201 of SEQ ID NO: 1 is substituted with Ser (201 Ser);

- position 205 of SEQ ID NO: 1 is substituted with Lys (205Lys); and/or
- position 255 of SEQ ID NO: 1 is substituted with Cys (255Cys).

In another embodiment the invention relates to a variant of a Glucose Dehydrogenase (GlucDH) derived from *Bacillus subtilis,* wherein said variant comprises an amino acid sequence having at least 90% identity to SEQ ID NO:1 and wherein said variant comprises a substitution of amino acid residue glutamic acid at a position corresponding to position 170 of SEQ ID NO: 1 with an amino acid residue lysine and a substitution of amino acid residue glutamine at a postion corresponding to position 252 of SEQ ID NO: 1 with an amino acid residue leucine, and wherein said variant comprises at least one additional amino acid substitution selected from the group consisting of a substitution of amino acid residue leucine at a position corresponding to position 95 of SEQ ID NO: 1 with an amino acid residue isoleucine or valine and a substitution of amino acid residue asparagine at a position corresponding to position 97 of SEQ ID NO: 1 with an amino acid residue serine, said variant optionally further comprising additional amino acid substitutions of amino acid residue tyrosine at a position corresponding to position 39 of SEQ ID NO: 1 with an amino acid residue glutamic acid and/or of amino acid residue serine at a position corresponding to position 40 of SEQ ID NO: 1 with an amino acid residue cysteine.

In some embodiments said variant comprises or consists of an amino acid sequence that has at least 95% sequence identity to SEQ ID NO:1.

In another aspect the invention relates to an isolated polynucleotide encoding the GlucDH variant protein disclosed above. In another aspect the invention relates to an expression vector comprising an isolated polynucleotide as defined above operably linked to a promoter sequence capable of promoting the expression of said polynucleotide in a host cell. In another aspect the invention relates to a host cell comprising the expression vector described above. In another aspect the invention relates to a process for producing GlucDH variants comprising culturing the host cell described above under conditions suitable for production of the enzyme variants.

In another aspect the invention relates to a method of detecting, determining or measuring glucose in a sample using a GlucDH variant described above, comprising contacting the sample with said variant. In some embodiments the detection, determination or measurement of glucose is performed using a sensor or test strip device.

In another aspect the invention relates to the use of a GlucDH variant described above for determining the amount or concentration of glucose in a sample.

In another aspect the invention relates to a device for the detection or measurement of glucose in a sample comprising a GlucDH variant described above and other reagents required for said measurement. In some embodiments, the device is or comprises a sensor or a test strip.

## DETAILED DESCRIPTION OF THE INVENTION

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which a solely and exclusively consists of B) and to a situation in which, besides B, one or more further elements are present in entity A, such as element C, elements C and D or even further elements.

As set out above, in a first embodiment the invention relates to a variant of a Glucose Dehydrogenase (GlucDH) derived from *Bacillus subtilis,* wherein said variant comprises an amino acid sequence having at least 90% identity to SEQ ID NO:1 and wherein said variant comprises a substitution of amino acid residue glutamic acid at a position corresponding to position 170 of SEQ ID NO: 1 with an amino acid residue lysine and a substitution of amino acid residue glutamine at a postion corresponding to position 252 of SEQ ID NO: 1 with an amino acid residue leucine, and wherein said variant further comprises one or more additional amino acid substitutions selected from the group consisting of a substitution of amino acid residue leucine at a position corresponding to position 95 of SEQ ID NO: 1 with an amino acid residue isoleucine or valine, a substitution of amino acid residue asparagine at a position corresponding to position 97 of SEQ ID NO: 1 with an amino acid residue serine, a substitution of amino acid residue glycine at a position corresponding to position 163 of SEQ ID NO: 1 with an amino acid residue alanine, a substitution of amino acid residue glutamic acid at a position corresponding to position 223 of SEQ ID NO: 1 with an amino acid residue phenylalanine, tryptophan, isoleucine, leucine, threonine or tyrosine, and a substitution of amino acid residue serine at a position corresponding to position 237 of SEQ ID NO: 1 with an amino acid residue glutamic acid, arginine or asparagine.

In one embodiment the invention relates to a variant of a Glucose Dehydrogenase (GlucDH) derived from Bacillus subtilis, said variant having at least 90% identity to SEQ ID NO:1 and comprising a substitution of amino acid residue glutamic acid at a position corresponding to position 170 of SEQ ID NO: 1 with an amino acid residue lysine

and a substitution of amino acid residue glutamine at a postion corresponding to position 252 of SEQ ID NO: 1 with an amino acid residue leucine, and wherein said variant further comprises one or more additional amino acid substitutions selected from the group consisting of a substitution of amino acid residue leucine at a position corresponding to position 95 of SEQ ID NO: 1 with an amino acid residue isoleucine or valine, a substitution of amino acid residue glycine at a position corresponding to position 163 of SEQ ID NO: 1 with an amino acid residue alanine, a substitution of amino acid residue glutamic acid at a position corresponding to position 223 of SEQ ID NO: 1 with an amino acid residue phenylalanine, tryptophan, isoleucine, leucine, threonine or tyrosine, and a substitution of amino acid residue serine at a position corresponding to position 237 of SEQ ID NO: 1 with an amino acid residue glutamic acid, arginine or asparagine.

The sequence of the wild-type glucose dehydrogenase from Bacillus subtilits is shown as SEQ ID NO: 1:

SEQ ID NO: 1 (GlucDH from *Bacillus subtilis*)

```
MYPDLKGKVV AITGAASGLG KAMAIRFGKE QAKVVINYYS NKQDPNEVKE EVIKAGGEAV    60
VVQGDVTKEE DVKNIVQTAI KEFGTLDIMI NNAGLENPVP SHEMPLKDWD KVIGTNLTGA   120
FLGSREAIKY FVENDIKGNV INMSSVHEVI PWPLFVHYAA SKGGIKLMTE TLALEYAPKG   180
IRVNNIGPGA INTPINAEKF ADPKQKADVE SMIPMGYIGE PEEIAAVAVW LASKESSYVT   240
GITLFADGGM TQYPSFQAGR G                                             261
```

With the introduction of mutations into the wild-type sequence (SEQ ID NO: 1), GlucDH variants are obtained. The variants are functionally active, i.e. they convert glucose to gluconolactone.

The GlucDH variant of the present invention comprises an amino acid sequence having at least 90 % identity to the amino acid sequence of SEQ ID NO: 1 (GlucDH from *Bacillus subtilis*).

The term "at least 90 % identical" or "at least 90 % identity" as used herein means that the sequence of the variant GlucDH according to the present invention has an amino acid sequence characterized in that, within a stretch of 100 amino acids, at least 90 amino acids residues are identical to the sequence of SEQ ID NO:1. Sequence identities of other percentages are defined accordingly.

Sequence identity according to the present invention can, e.g., be determined by methods of sequence alignment in form of sequence comparison. Methods of sequence alignment are well known in the art and include various programs and alignment algorithms which have been described in, e.g., Pearson and Lipman (1988). Moreover, the NCBI Basic Local Alignment Search Tool (BLAST) is available from several sources, including the National Center for Biotechnology Information (NCBI, Bethesda, MD) and on the internet, for use in connection with the sequence analysis programs blastp, blastn, blastx, tblastn and tblastx. Percentage of identity of variants according to the present invention relative to the amino acid sequence of e.g. SEQ ID NO: 1 is typically characterized using the NCBI Blast blastp with standard settings. Alternatively, sequence identity may be determined using the software GENEious with standard settings. Alignment results can be, e.g., derived from the Software Geneious (version R8), using the global alignment protocol with free end gaps as alignment type, and Blosum62 as a cost matrix. In one embodiment, the GlucDH variant of the present invention comprises the additional amino acid substitutions of amino acid residue glutamic acid at the position corresponding to position 223 of SEQ ID NO: 1 with an amino acid residue phenylalanine, tryptophan, isoleucine, leucine, threonine or tyrosine, and of amino acid residue serine at the position corresponding to position 237 of SEQ ID NO: 1 with an amino acid residue glutamic acid, arginine or asparagine. In one embodiment, the GlucDH variant of the present invention futher comprises the additional amino acid substitutions at the position corresponding to position 163 of SEQ ID NO:1 with an amino acid residue alanine.

It could be shown that the indicated substitutions can improve the performance of the enzyme. The indicated substitution at position 95, 97, 163, 223 and/or 237 are particularly suitable in order to increase affinity for the glucose and for cNAD, as shown in Tables 1 and 2 for single and multiple mutations.

In one embodiment, the GlucDH variant of the present invention further comprises one or more additional amino acid substitutions, wherein the amino acid at the position corresponding to

- position 39 of SEQ ID NO: 1 is substituted with Glu (39Glu);
- position 40 of SEQ ID NO: 1 is substituted with Cys (40Cys);
- position 46 of SEQ ID NO: 1 is substituted with Asp (46Asp);
- position 70 of SEQ ID NO: 1 is substituted with Cys (70Cys);
- position 78 of SEQ ID NO: 1 is substituted with Ala (78Ala);

- position 80 of SEQ ID NO: 1 is substituted with Leu (80Leu);
- position 96 of SEQ ID NO: 1 is substituted with Leu (96Leu),Gln (96Gln), Val (96Val), or Met (96Met);
- position 107 of SEQ ID NO: 1 is substituted with Glu (107Glu);
- position 134 of SEQ ID NO: 1 is substituted with Glu (134Glu);
- position 178 of SEQ ID NO: 1 is substituted with Ser (178Ser);
- position 201 of SEQ ID NO: 1 is substituted with Ser (201 Ser);
- position 205 of SEQ ID NO: 1 is substituted with Lys (205Lys); and/or
- position 255 of SEQ ID NO: 1 is substituted with Cys (255Cys).

Thereby, the performance of the enzyme can be further impoved (see Table 2).

In another embodiment, the GlucDH variant of the present invention, comprises at least one additional amino acid substitution selected from the group consisting of the substitution of amino acid residue leucine at the position corresponding to position 95 of SEQ ID NO: 1 with an amino acid residue isoleucine or valine and the subsitution of amino acid residue asparagine at the position corresponding to position 97 of SEQ ID NO: 1 with an amino acid residue serine, said variant optionally further comprising additional amino acid substitutions of amino acid residue tyrosine at the position corresponding to position 39 of SEQ ID NO: 1 with an amino acid residue glutamic acid and/or of amino acid residue serine at the position corresponding to position 40 of SEQ ID NO: 1 with an amino acid residue cysteine.

The GlucDH variant of the present invention may comprise other substitutions than those mentioned above and/or deletions and/or insertions provided that it comprises an amino acid sequence that is at least 90 % identical to the amino acid sequence of SEQ ID NO: 1.

The GlucDH variant of the present invention in one embodiment consists of an amino acid sequence having at least 90% identity to SEQ ID NO: 1. In another embodiment, the variant comprises or consists of an amino acid sequence that has at least 95 % identity to the amino acid sequence of SEQ ID NO: 1. In one embodiment, the variant GlucDH comprises or consists of an amino acid sequence that has at least 96 % or 97 % identity to the amino acid sequences of SEQ ID NO: 1. Sequence identity may be determined as described above.

In one embodiment of the present invention, the GlucDH variant of the present invention comprises or consists of an amino acid sequence that has has at least 95 %, 96 %, 97 %, 98 %, or 99 % identity to the amino acid sequence of any of SEQ ID NOs: 2-5. In another embodiment the GlucDH variant of the present inventions consists of the sequence selected from the group consisting of SEQ ID NOs: 2-5. The sequences of SEQ ID NOs: 2-5 are shown in the section "Sequences". Sequence identity may be determined as described above.

The present invention also relates to an isolated polynucleotide encoding the GlucDH variant protein of the present invention. The term "nucleic acid" as used herein generally relates to any nucleotide molecule which encodes the Gluc DH variant of the invention and which may be of variable length. Examples of a nucleic acid of the invention include, but are not limited to, plasmids, vectors, or any kind of DNA and/or RNA fragment(s) which can be isolated by standard molecular biology procedures, including, e.g. ion-exchange chromatography. A nucleic acid of the invention may be used for transfection or transduction of a particular cell or organism.

Nucleic acid of the present invention may be in the form of RNA, such as mRNA or cRNA, or in the form of DNA, including, for instance, cDNA and genomic DNA e.g. obtained by cloning or produced by chemical synthetic techniques or by a combination thereof. The DNA may be triple-stranded, double- stranded or single-stranded. Single-stranded DNA may be the coding strand, also known as the sense strand, or it may be the non-coding strand, also referred to as the anti-sense strand. Nucleic acid as used herein also refers to, among other, single- and double- stranded DNA, DNA that is a mixture of single- and double-stranded RNA, and RNA that is a mixture of single- and double-stranded regions, hybrid molecules comprising DNA and RNA that may be single-stranded or, more typically, double-stranded, or triple-stranded, or a mixture of single- and double-stranded regions. In addition, nucleic acid as used herein refers to triple-stranded regions comprising RNA or DNA or both RNA and DNA. Additionally, the nucleic acid may contain one or more modified bases. Moreover, DNAs or RNAs comprising unusual bases, such as inosine, or modified bases, such as tritylated bases, to name just two examples, are nucleic acids within the context of the present invention. The term nucleic acid as it is employed herein embraces chemically, enzymatically or metabolically modified forms of nucleic acid molecule, as well as the chemical forms of DNA and RNA characteristic of viruses and cells, including simple and complex cells, inter alia.

The nucleic acid of the invention may be originally formed *in vitro* or in a cell in culture, in general, by the

manipulation of nucleic acids by endonucleases and/or exonucleases and/or polymerases and/or ligases and/or recombinases or other methods known to the skilled practitioner to produce the nucleic acids.

The present invention further relates to an expression vector comprising the isolated polynucleotide operably linked to a promoter sequence capable of promoting the expression of said polynucleotide in a host cell. As used herein, the term "expression vector" generally refers to any kind of nucleic acid that can be used to express a protein of interest in a cell (see also above details on the nucleic acids of the present invention). In particular, the expression vector of the invention can be any plasmid or vector known to the person skilled in the art which is suitable for expressing a protein in a particular host cell including, but not limited to, mammalian cells, bacterial cell, and yeast cells. An expression construct of the present invention may also be a nucleic acid which encodes a GlucDH variant of the invention, and which is used for subsequent cloning into a respective vector to ensure expression. Plasmids and vectors for protein expression are well known in the art, and can be commercially purchased from diverse suppliers including, e.g., Promega (Madison, WI, USA), Qiagen (Hilden, Germany), Invitrogen (Carlsbad, CA, USA), or MoBiTec (Germany). Methods of protein expression are well known to the person skilled in the art and are, e.g., described in Sambrook et al., 2000 (Molecular Cloning: A laboratory manual, Third Edition).

The vector may additionally include nucleic acid sequences that permit it to replicate in the host cell, such as an origin of replication, one or more therapeutic genes and/or selectable marker genes and other genetic elements known in the art such as regulatory elements directing transcription, translation and/or secretion of the encoded protein. The vector may be used to transduce, transform or infect a cell, thereby causing the cell to express nucleic acids and/or proteins other than those native to the cell. The vector optionally includes materials to aid in achieving entry of the nucleic acid into the cell, such as a viral particle, liposome, protein coating or the like. Numerous types of appropriate expression vectors are known in the art for protein expression, by standard molecular biology techniques. Such vectors are selected from among conventional vector types including insects, e.g., baculovirus expression, or yeast, fungal, bacterial or viral expression systems. Other appropriate expression vectors, of which numerous types are known in the art, can also be used for this purpose. Methods for obtaining such expression vectors are well-known (see, e.g. Sambrook et al, supra).

As detailed above, the nucleic acid which encodes GlucDH variant of the invention is operably linked to sequence which is suitable for driving the expression of a protein in a host cell, in order to ensure expression of the protein. However, it is encompassed within the present invention that the claimed expression construct may represent an intermediate product, which is subsequently cloned into a suitable expression vector to ensure expression of the protein. The expression vector of the present invention may further comprise all kind of nucleic acid sequences, including, but not limited to, polyadenylation signals, splice donor and splice acceptor signals, intervening sequences, transcriptional enhancer sequences, translational enhancer sequences, drug resistance gene(s) or alike. Optionally, the drug resistance gene may be operably linked to an internal ribosome entry site (IRES), which might be either cell cycle-specific or cell cycle-independent.

The term "operably linked" as used herein generally means that the gene elements are arranged as such that they function in concert for their intended purposes, e.g. in that transcription is initiated by the promoter and proceeds through the DNA sequence encoding the protein of the present invention. That is, RNA polymerase transcribes the sequence encoding the fusion protein into mRNA, which in then spliced and translated into a protein.

The term "promoter sequence" as used in the context of the present invention generally refers to any kind of regulatory DNA sequence operably linked to a downstream coding sequence, wherein said promoter is capable of binding RNA polymerase and initiating transcription of the encoded open reading frame in a cell, thereby driving the expression of said downstream coding sequence. The promoter sequence of the present invention can be any kind of promoter sequence known to the person skilled in the art, including, but not limited to, constitutive promoters, inducible promoters, cell cycle-specific promoters, and cell type-specific promoters.

Furthermore, the present invention also relates to a host cell comprising the expression vector of th present invention. The cell is preferably a host cell. A "host cell" of the present invention can be any kind of organism suitable for application in recombinant DNA technology, and includes, but is not limited to, all sorts of bacterial and yeast strain which are suitable for expressing one or more recombinant protein(s). Examples of host cells include, for example, various *E. coli* strains. A variety of *E. coli* bacterial host cells are known to a person skilled in the art and include, but are not limited to, strains such as DH5-alpha, HB101, MV1190, JM109, JM101, or XL-1 blue which can be commercially purchased from diverse suppliers including, e.g., Stratagene (CA, USA), Promega (WI, USA) or Qiagen (Hilden, Germany). A particularly suitable host cell is also described in the Examples, namely *E. coli* XL-1Blue cells.

The present invention also relates to a process for producing GlucDH variants comprising culturing the host cell under conditions suitable for production of the enzyme variants. The cultivation of host cells according to the invention is a routine procedure known to the skilled person. That is, a nucleic acid encoding a GlucDH variant of the invention can be introduced into a suitable host cell(s) to produce the respective protein by recombinant means. These host cells can by any kind of suitable cells, preferably bacterial cells such as *E. coli,* which can be easily cultivated. At a first step, this approach may include the cloning of the respective gene into a suitable plasmid vector. Plasmid vectors are widely used for gene cloning, and can be easily introduced, i.e. transformed, into bacterial cells which have been made competent. After the protein has been expressed in the respective host cell, the cells can be broken by means of either chemical or mechanical cell lysis are well known to the person skilled in the art, and include, but are not limited to, e.g. hypotonic salt treatment, detergent treatment, homogenization, or ultrasonification.

In another aspect the present invention relates to a method of detecting, determining or measuring glucose in a sample using a GlucDH variant according to the present invention, comprising contacting the sample with said variant. The method may be performed using a sensor or test strip device.

More particularly, the method of determining glucose in a sample may comprise

a) contacting the sample with the GlucDH variant of the present invention under conditions conducive to the activity of the GlucDH

b) reacting glucose with carba nicotinamide adenine dinucleotide (cNAD) or a functionally active derivative thereof; and

c) determining the change in the redox state of cNAD or the derivative thereof,

thereby determining the amount or concentration of glucose in the sample.

The above method is based on the fact that GlucDH may be used to catalyze the conversion of glucose to gluconolactone according to the following scheme:

$$glucose + \mathrm{cNAD}^{+} \rightleftharpoons \mathrm{gluconolactone} + \mathrm{cNADH} + \mathrm{H}^{+}$$

In carba-NAD (cNAD) the ribose is substituted by a carbacyclic sugar unit compared to NAD. Carba-NAD has the following structure (I):

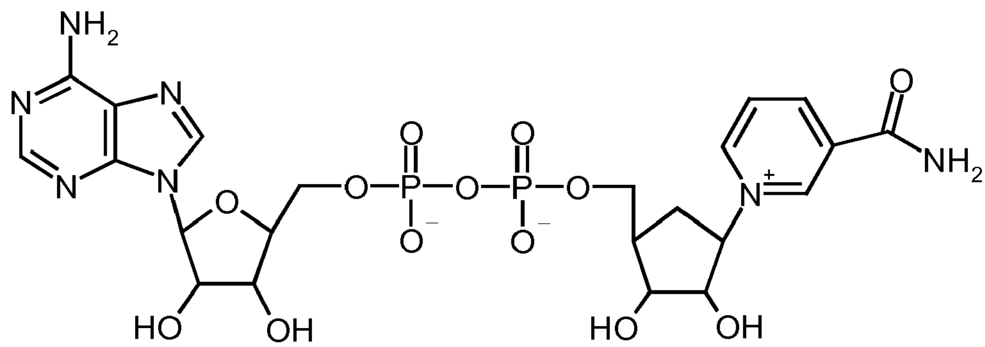

(I)

The compound, its production and use are described in detail in WO 2007/012494, WO 2011/012270 and WO2014/195363. The cofactor in the present invention is preferably carba-NAD. In one embodiment of the present invention, a cNAD or a functionally active derivative of cNAD is used as disclosed in formula III of WO 2011/012270 to which it is explicitly referred. In one embodiment of the present invention, cNADP is used instead of cNAD.

In a first step of the method of the present invention a sample is contacted with the GlucDH variant of the present invention. The contacting of the sample with the GluDH can be direct (e.g. in liquid assays) or indirect (e.g. in sensor systems in which only a fraction of the sample (containing the analyte) is contacting the GlucDH. It is evident that the contacting should be carried out under conditions conducive to the activity of the GlucDH variant, i.e. allowing the enzyme to convert glucose to gluconolactone. Incubation step can vary from about 3 seconds to several hours, preferably from about 3 seconds to about 10 minutes. However, the incubation time will depend upon the assay format, volume of solution, concentrations and the like. Usually the assay will be carried out at ambient temperature or a temperature required for other test formats carried out concomitantly (e.g. 25°C to 38°C; such as 30°C or 37 °C), although it can be conducted over a range of temperatures, such as 10°C to 40°C.

Optionally, the enzyme can be fixed to or immobilized into a support layer prior to the contacting with the sample to facilitate the assay. Examples of support layers include glass or plastic in the form of, for example, a microtiter plate, a glass microscope slide or cover slip, a stick, a bead, or a microbead, membranes (e.g. used in test strips) and layers of biosensors.

The sample may be any sample suspected of containing glucose, particularly a sample from a subject. The

term "sample from a subject" includes all biological fluids, excretions and tissues isolated from any given subject, particularly a human. In the context of the present invention such samples include, but are not limited to, blood, blood serum, blood plasma, nipple aspirate, urine, semen, seminal fluid, seminal plasma, prostatic fluid, excreta, tears, saliva, sweat, biopsy, ascites, cerebrospinal fluid, milk, lymph, bronchial and other lavage samples, or tissue extract samples. Preferably, the subject is an animal (including human), more preferably a mammal, still more preferably a human. Preferably, the sample is a body fluid, particularly a blood sample or a urine sample.

Typically, blood samples are preferred test samples for use in the context of the present invention.

After the contacting and the conversion of glucose, if present, the change in the redox state of cNAD or derivate mediated by the GlucDH variant are determined, thereby determining glucose in the sample. Evidently, the amount of cNADH or derivate thereof produced and the amount of cNAD or derivate thereof consumed correlate with the amount of glucose present in the sample. Accordingly, the change in the redox state of cNAD includes the determination of the amount or concentration of cNAD and/or cNADH as well as the ratio of the two. The same applies to cNAD derivates.

A variety of methods for determining cNADH/NAD or derivate thereof are known in the art and any of these can be used.

Exemplary methods for determining cNADH/NAD or derivate thereof include electrochemical methods (e.g. as described in US 6,541,216) or optical methods (e.g. by measuring cNAD/cNADH conversion by light absorbance at e.g. 340 nm or 365 nm or by assays based on a reductase to form luciferin, which is then quantified optically). If electrochemical methods are used, cNADH/cNAD or derivate thereof can either a) react directly on a measurement electrode or b) cNADH/cNAD or derivate thereof reacts in a first step with an additional redoxmediator substance which changes its redox state in a defined relation to the redox state of cNADH/cNAD or derivate thereof and this redoxmediator reacts in a subsequent step on the measurement electrode.

The method of the present invention can be carried out in a so-called liquid or wet test, for example in a cuvette, or as a so-called dry test on an appropriate reagent carrier, the necessary test reagents thereby being present in or on a solid carrier, which is preferably an absorbent or swellable material.

Alternatively or additionally, the GlucDH may be part of a sensor, a test strip, a test element, a test strip device or a liquid test.

A sensor is an entity that measures a physical/chemical quantity and converts it into a signal which can be read by an observer or by an instrument. In the present invention, the GlucDH may be part of a sensor. The sensor converts glucose and cNAD or a derivate thereof into gluconolactone and cNADH or a derivate thereof, which is further converted into a signal such as a change in colour or a value displayed e.g. on a display or monitor.

In one embodiment, the sensor may comprise GlucDH and an amperometric device to determine glucose of a sample. Enzyme-coupled biosensors have been described in the art. In accordance with this, GlucDH may be coupled to a surface (e.g. by printing a GlucDH / graphite mixture onto electroplated graphite pads or by adsorption or immobilization of the GlucDH on carbon particles, platinized carbon particles, carbon/manganese dioxide particles, glassy carbon, or mixing it with carbon paste electrodes etc.)

A test strip or a test element is an analytic or diagnostic device used to determine presence and/or quantity of a target substance within a sample. A standard test strip may comprise one or more different reaction zones or pads comprising reagents which react (e.g. change colour) when contacted with a sample. Test strips are known in many embodiments, for example from US 6,541,216, EP 262445 and US 4816224. It is commonly known that one or more reagents (e.g. enzymes) needed for carrying out the determination methods are present on or in solid carrier layers. As carrier layers, there are especially preferred absorbent and/or swellable materials which are wetted by the sample liquid to be analyzed. Examples include gelatine, cellulose and synthetic fiber fleece layers.

The GlucDH of the present invention may also be part of a liquid test. A liquid test is a test wherein test components react in a liquid medium. Usually in the field of laboratory analytics, the liquid reagents are on water basis, e.g. a buffered salt solution in order to provide the activity of enzyme(s) involved. The liquid is usually adapted to the specific intended use. For carrying out a liquid test, all test components are solved in a liquid and combined (or vice versa). Typical containments for carrying out such tests include vials, multi wells plates, cuvettes, vessels, reagent cups, tubes etc.

Accordingly, the method of the present invention may further be characterized in that

a) wherein the determining the change in the redox state of cNAD or the derivate thereof includes the determination of the concentration of (i) cNAD or the derivate thereof and/or (ii) cNADH or the derivate thereof; and/or

b) wherein the determining the change in the redox state of cNAD or the derivative thereof is electrochemically or optically; and/or

c) wherein the method further comprises determining the amount or concentration of gluconolactone; and/or

d) wherein the GlucDH variant is part of a sensor, a test strip, a test element, a test strip device or a liquid test; and/or

e) wherein the sample is a body fluid, particularly a blood sample or a urine sample.

With respect to the use of the present invention it is referred to the terms, examples and specific embodiments used in the context of the other aspects of the present disclosure, which are also applicable to this aspect. For details it may be referred to the methods of the present invention.

Yet, in another aspect, the present invention relates to a device for the detection or measurement of glucose in a sample comprising a GlucDH of the present invention and other reagents required for said measurement. With respect to the device of the present invention it is referred to the terms, examples and specific embodiments used in the context of the other aspects of the present disclosure, which are also applicable to this aspect.

The device may be or comprise a sensor, preferably an electrochemical sensor or an optical sensor, or a test strip, particularly a test strip.

A sensor is an analytical device for the detection of an analyte that combines a biological component (here the glucose according to the present invention) with a detector component, particularly a physicochemical detector component. Exemplary sensors based on an electrochemical test strip format are described in U.S. Patent Nos. 5,413,690; 5,762,770 and 5,997,817.

An electrochemical sensor is based on the translation of a chemical signal (here presence of glucose) into an electrical signal (e.g. current). A suitable electrode can measure the glucose mediated production of cNADH or derivative thereof as an electrical signal. A suitable optical sensor can measure the GlucDH-mediated change in the redox state of cNAD or derivate thereof The signal may be the cNAD/cNADH-mediated absorbance / emission of light.

The device of the present invention may comprise - in addition to the GlucDH of the present invention - one or more further component(s), such as other reagents, required for or helpful in said determining. The components may be any of these described in the context of the methods and devices of the present invention. Additionally, this may include an instruction manual, a lancet device, a capillary pipette, a further enzyme, a substrate and/or a control solution etc.

Unless defined otherwise, all technical and scientific terms and any acronyms used herein have the same meanings as commonly understood by one of ordinary skill in the art in the field of the invention. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1 -56081 -569-8).

The invention is not limited to the particular methodology, protocols, and reagents described herein because they may vary. Although any methods and materials similar or equivalent to those described herein can be used in the practice of the present invention, the preferred methods, and materials are described herein. Further, the terminology used herein is for the purpose of describing particular embodiments only and is not intended to limit the scope of the present invention.

In the following examples, all reagents, restriction enzymes, and other materials were obtained from Roche Diagnostics Germany, unless other commercial sources are specified, and used according to the instructions given by the suppliers. Operations and methods employed for the purification, characterization and cloning of DNA are well known in the art (Ausubel, F., et al., in "Current protocols in molecular biology" (1994) Wiley Verlag) and can be adapted as required by the skilled artisan.

The following examples further illustrate the present invention. These examples are not intended to limit the scope of the present invention, but provide further understanding of the invention.

## EXAMPLES

### Example 1

### ***First Round of Mutagenesis***

Wild type Glucose Dehydrogenase from *Bacillus subtilis* was mutated at positions 170 and 252 (i.e. E170K and Q252L) in order to improve thermal stability. This mutant is referred further as GlucDH Mut.A. For improving enzymatic activity of this GlucDH variant carrying mutations E170K and Q252L in the presence of the artificial cofactor cNAD a first round of mutagenesis was applied.

QuikChange Site-Directed Mutagenesis Kit (Stratagene, Cat. 200518) was used to substitute successively wild type amino acids at defined positions of the enzyme.

Hot spot regions were identified by analysis of x-ray structures of the enzyme from Bacillus megaterium. The

corresponding positions were mutated by saturation mutagenesis.

The 5'- and the 3'-primer used for mutagenesis were complementary to each other and contained NNN (randomly synthesized nucleotides) for the amino acid exchange in a central position. This randomly created codon was flanked by 12 to 16 nucleotides at each end. The sequences of these nucleotides were identical to the cDNA-strand or to the complementary cDNA-strand flanking the codon for the amino substitution. Mutant library was created by transformation of mutated genes in *E.coli* strain Xl-Blue and cultivation on agar plates over night at 37°C.

Example 2

***Determination of Properties of Variant GlucDHs from First Round of Mutagenesis***

GlucDH variants 1-18 obtained as described in Example 1 were analyzed for their enzymatic properties (i.e., thermal stability, activity with cofactor cNAD, KM value for glucose and substrate specificity). The results relative to GlucDH variant carrying mutations E170K and Q252L are summarized in Table 1 (+ = improved; o = similar; - = decreased).

The clones obtained in Example 1 were cultivated in LB media, cell disrupted and the enzymatic activity of glucose dehydrogenase determined. To identify the different properties of the mutated enzymes the following procedure was applied:

- Reference Measurement:

  Measurement under standard conditions with NAD and Glucose in saturated concentrations for the enzyme

- Affinity to Glucose:

  Measurement under glucose limitation => comparison to reference measurement => higher values indicate better km values

- Thermal Stability:

  Incubation of sample at elevated temperature for 30 Min => comparison to reference measurement => higher remaining activity = better temperature stability

- cNAD acceptance:

  Measurement with cNAD and comparison to reference measurement => higher values = better enzymatic activity with cNAD

- Substrate specificity:

  Measurement with xylose instead of glucose as substrate => comparison to reference measurement => statement to substrate specificity => lower values= better specificity to glucose

In detail, samples were prepared as follows:

Mutant colonies on agar plates described in example 1 were picked in microtiter plates (mtp) containing 200 µl LB-Ampicillin-media/hole and incubated at 37°C over night.

These plates were referred to as master plates. For each amino acid position two master plates were picked to assure that every possible exchange is included.

From each master plate 20 µl of cultivated clone/cavity was transferred to a mtp containing 250 µl 0.14 % Triton X-100, 190 mM NaCl, 4.8% B-PER (Thermo Scientific Prod.78248), 95 mM Tris pH 8.8/cavity and incubated for cell disruption at 50°C for 30 minutes. This plate was referred to as working plate.

From the working plate 5 x 10µl sample / cavity were transferred to five empty mtps.

With one mtp a standard measurement was performed with 90 µl reagent solution containing 0.11 % Triton X-100, 145 mM NaCl,1.37 mM NAD, 200 mM glucose, 73 mM Tris pH8.8 (reference measurement).

With another mtp measurement under glucose limitation was performed with with 90 µl reagent solution containing 0.11 % Triton X-100, 145 mM NaCl,1.37 mM NAD, 12.5 mM glucose, 73 mM Tris pH8.8.

With another mtp temperature stability was examined by determination of remaining activity after 30 minutes

incubation at 80°C with standard test conditions as described above.

With another mtp cNAD acceptance was measured with 90 µl reagent solution containing 1.37 mM cNAD, 0.11 % Triton X-100, 145 mM NaCl, 200 mM glucose, 73 mM Potassium phosphate pH7.0.

With another mtp Xylose conversion was measured with 90 µl reagent solution containing 0.11 % Triton X-100, 145 mM NaCl,1.37 mM NAD, 1M xylose, 73 mM Tris pH8.8.

The enzymatic reaction was monitored at room temperature at 340 nm for 5 minutes and the dE/min calculated for each working plate. The value from the reference measurement was set to 100% activity. The values obtained with the other four plates were compared to the reference measurement and calculated in percent activity ((dE/min Parameter/dE/min Reference)* 100).

Calculation of different screening parameters:

Affinity to glucose (expressed as activity ratio) was calcualted as follows:

$$\left(\frac{\text{dE/min obtained with less glucose}}{\text{dE/min obtained with glucose in saturation}}\right) * 100 = \text{activity in percent}$$

Thermal Stability (expressed as remaining activity) was calculated as follows:

$$\left(\frac{\frac{\text{dE}}{\text{min}}\text{stressed sample (i.e. in example 2 30 min 80°C}}{\frac{\text{dE}}{\text{min}}\text{not stressed sample}}\right) * 100$$

$$= \text{remaining activity in percent}$$

cNAD acceptance (expressed as activity ratio) was calculated as follows:

$$\left(\frac{\text{dE/min obtained with cNAD}}{\text{dE/min obtained with NAD}}\right) * 100 = \text{activity in percent}$$

Substrate specificity (expressed as activity ratio) was calculated as follows:

$$\left(\frac{\text{dE/min obtained with Xylose}}{\text{dE/min obtained with glucose in saturation}}\right) * 100 = \text{activity in percent}$$

Summary of Table1 below:

Variant 18 was valuated the best mutant for further improvement since it has the best cNAD acceptance and affinity for glucose. Throwback is its low temperature stability. This mutant is referred further as GlucDH Mut.B.

Table 1

| Variant | Substitutions | Additional Substitutions | | | | Thermal Stability | Activity with Cofactor cNAD | KM Value for Glucose | Substrate Specificity |
|---|---|---|---|---|---|---|---|---|---|
| 1 | E170K + Q252L | L95I | | | | ++ | + | +- | - |
| 2 | E170K + Q252L | L95I | N97S | | | ++ | + | + | - |
| 3 | E170K + Q252L | L95I | N97S | Y39E | S40C | ++ | ++ | ++ | - |
| 4 | E170K + Q252L | | N97S | Y39E | S40C | ++ | ++ | ++ | - |
| 5 | E170K + Q252L | L95V | | | | + | + | + | - |
| 6 | E170K + Q252L | | G163A | | | - | ++ | + | - |
| 7 | E170K + Q252L | | | E223W | | + | + | ++ | - |
| 8 | E170K + Q252L | | | E223F | | +/o | + | + | o |
| 9 | E170K + Q252L | | | | S237N | + | + | ++ | o |
| 10 | E170K + Q252L | | | | S237R | +/o | + | + | o |
| 11 | E170K + Q252L | | | | S237E | - | + | + | - |
| 12 | E170K + Q252L | | | E223I | S237N | + | + | + | o |
| 13 | E170K + Q252L | | | E223L | S237N | O | + | + | o |
| 14 | E170K + Q252L | | | E223F | S237N | + | + | + | o |
| 15 | E170K + Q252L | | | E223T | S237N | + | + | + | - |
| 16 | E170K + Q252L | | | E223Y | S237N | + | + | + | o |
| 17 | E170K + Q252L | | | E223W | S237N | + | + | + | - |
| 18 | E170K + Q252L | | G163A | E223W | S237N | - | +++ | ++ | o |

(+ = improved; o = similar; - = decreased)

## Example 3

### *Second Round of Mutagenesis*

Based on GlucDH variant 18 (see Table 1) carrying mutations E170K, Q252L, G163A, E223W and S237N = GlucDH Mut.B a further round of mutagenesis was carried out in order to enhance thermal stability, while maintaining or further improving specific activity, affinity for glucose using cNAD as artificial cofactor and substrate specificity.

Exchanges found during mutagenesis of GlucDH Mut.A concerning temperature stability were applied for GlucDH Mut.B.

Mutagenesis was performed as described above.

## Example 4

### *Determination of Properties of Variant GlucDHs from Second Round of Mutagenesis*

GlucDH variants 19 - 50 obtained as described in Example 3 were analyzed for their enzymatic properties (i.e., thermal stability, KM value for glucose and substrate specificity). The results relative to variant 18 (GlucDHMut.B) carrying mutations E170K, Q252L, G163A, E223W and S237N are summarized in Table 2 (+ = improved; o = similar; - = decreased).

The clones obtained in Example 3 were cultivated in LB media, cell disrupted and the enzymatic activity of glucose dehydrogenase determined. To identify the different properties of the mutated enzymes the following procedure was applied:

The screening system was changed compared to example 2 concerning cofactor appliance:

- Reference Measurement:

  Measurement under standard conditions with cNAD and Glucose in saturated concentrations for the enzyme

- Affinity to Glucose:

  Measurement under glucose limitation => comparison to reference measurement => higher values indicate better km values

- Thermal Stability:

  Incubation of sample at elevated temperature for 30 Min => comparison to reference measurement => higher remaining activity = better temperature stability

- Substrate specificity:

  Measurement with xylose instead of glucose as substrate => comparison to reference measurement => statement to substrate specificity => lower values indicate better specificity to glucose

In detail, samples were prepared as described in example 3 to obtain master and working plates. From the working plate 4 x 10μl sample / cavity were transferred to four empty mtps.

With one mtp a standard measurement was performed with 90 μl reagent solution containing 0.11 % Triton X-100, 145 mM NaCl,1.37 mM cNAD, 200 mM glucose, 73 mM Tris pH8.0, (reference measurement).

With another mtp measurement under glucose limitation was performed with 90 μl reagent solution containing 0.11 % Triton X-100, 145 mM NaCl,1.37 mM cNAD, 12.5 mM glucose, 73 mM Tris pH8.0.

With another mtp temperature temperature stability was examined by determination of remaining activity after 30 minutes incubation at 68°C with standard test conditions as described above.

With another mtp Xylose conversion was measured with 90 μl reagent solution containing 0.11 % Triton X-100, 145 mM NaCl,1.37 mM cNAD, 1M xylose, 73 mM Tris pH8.0.

The enzymatic reaction was monitored at room temperature at 340 nm for 5 minutes and the dE/min calculated for each working plate. The value from the reference measurement was set to 100% activity. The values obtained with the other plates were compared to the refernece measurement and calculated in percent activity ((dE/min Parameter/dE/min Reference) * 100).

Calculation of different screening parameters:

Affinity to glucose (expressed as activity ratio) was calcualted as follows:

$$\left(\frac{\text{dE/min obtained with less glucose}}{\text{dE/min obtained with glucose in saturation}}\right) * 100 = \text{activity in percent}$$

Thermal Stability (expressed as remaining activity) was calculated as follows:

$$\left(\frac{\frac{\text{dE}}{\text{min}}\text{stressed sample (i.e. in example 4 30 min 68°C}}{\frac{\text{dE}}{\text{min}}\text{not stressed sample}}\right) * 100 = \text{remaining activity in percent}$$

Substrate specificity (expressed as activity ratio) was calculated as follows:

$$\left(\frac{\text{dE/min obtained with Xylose}}{\text{dE/min obtained with glucose in saturation}}\right) * 100 = \text{activity in percent}$$

Summary of Table 2 below:

Variant 34 from the mutants listed in Table 2 was judged as one of the best candidates for further examination. Besides its improved properties concerning KM value for Glucose, and thermal stability, the applied exchanges did not contain a Cystein which could potentially react with some reagents on a test strip.

TABLE 2

| Variant | Substitutions | Additional Substitutions | | | | Thermal Stability | KM Value for Glucose | Substrate Specificity |
|---|---|---|---|---|---|---|---|---|
| 19 | E170K + Q252L + G163A + E223W + S237N | Y39E | | | | + | o | o |
| 20 | E170K + Q252L + G163A + E223W + S237N | Y39E | S40C | | | + | o | o |
| 21 | E170K + Q252L + G163A + E223W + S237N | Y39E | | N46D | | + | + | o |
| 22 | E170K + Q252L + G163A + E223W + S237N | Y39E | S40C | N46D | | ++ | ++ | -- |
| 23 | E170K + Q252L + G163A + E223W + S237N | Y39E | S40C | L95I | | + | + | o |
| 24 | E170K + Q252L + G163A + E223W + S237N | Y39E | S40C | | Q205K | ++ | ++ | - |
| 25 | E170K + Q252L + G163A + E223W + S237N | Y39E | S40C | L95I | Q205K | o | ++ | -- |

(continued)

| Variant | Substitutions | Additional Substitutions | | | Thermal Stability | KM Value for Glucose | Substrate Specificity |
|---|---|---|---|---|---|---|---|
| 26 | E170K + Q252L + G163A + E223W + S237N | S40C | | | + | o | o |
| 27 | E170K + Q252L + G163A + E223W + S237N | S40C | Q205K | | ++ | o | + |
| 28 | E170K + Q252L + G163A + E223W + S237N | E70C | | | o | ++ | - |
| 29 | E170K + Q252L + G163A + E223W + S237N | E70C | P178S | | + | + | o |
| 30 | E170K + Q252L + G163A + E223W + S237N | E70C | P178S | E96L | ++ | ++ | -- |
| 31 | E170K + Q252L + G163A + E223W + S237N | T78A | | | + | o | o |
| 32 | E170K + Q252L + G163A + E223W + S237N | I80L | | | + | o | o |
| 33 | E170K + Q252L + G163A + E223W + S237N | I80L | L95I | | o | + | - |
| 34 | E170K + Q252L + G163A + E223W + S237N | I80L | L95I | Y39E | + | ++ | - |
| 35 | E170K + Q252L + G163A + E223W + S237N | I80L | L95I | S40C | + | + | o |
| 36 | E170K + Q252L + G163A + E223W + S237N | I80L | L95I | P178S | + | + | - |
| 37 | E170K + Q252L + G163A + E223W + S237N | I80L | L95I | A201S | ++ | ++ | - |
| 38 | E170K + Q252L + G163A + E223W + S237N | I80L | L95I | Q205K | o | + | - |
| 39 | E170K + Q252L + G163A + E223W + S237N | I80L | L95I | S255C | ++ | + | - |
| 40 | E170K + Q252L + G163A + E223W + S237N | | E96Q | | ++ | ++ | --- |
| 41 | E170K + Q252L + G163A + E223W + S237N | | E96V | | ++ | ++ | - |

(continued)

| Variant | Substitutions | Additional Substitutions | | | Thermal Stability | KM Value for Glucose | Substrate Specificity |
|---|---|---|---|---|---|---|---|
| 42 | E170K + Q252L + G163A + E223W + S237N | E96M | | | ++ | ++ | -- |
| 43 | E170K + Q252L + G163A + E223W + S237N | K107E | | | + | o | o |
| 44 | E170K + Q252L + G163A + E223W + S237N | N134E | | | + | o | o |
| 45 | E170K + Q252L + G163A + E223W + S237N | N134E | Q205K | | + | + | + |
| 46 | E170K + Q252L + G163A + E223W + S237N | P178S | | | o | + | - |
| 47 | E170K + Q252L + G163A + E223W + S237N | A201S | | | - | + | o |
| 48 | E170K + Q252L + G163A + E223W + S237N | Q205K | | | + | o | o |
| 49 | E170K + Q252L + G163A + E223W + S237N | Q205K | | S255C | + | o | + |
| 50 | E170K + Q252L + G163A + E223W + S237N | S255C | | | + | o | o |
| (+ = improved; o = similar; - = decreased) | | | | | | | |

**Example 5**

***Determination of Properties of Exemplary Variant GlucDHs***

Exemplary GlucDH variant from the first round of mutagenesis (GlucDH variant 2) and from the second round of mutagenesis (GlucDH variant 34) were further analyzed for their enzymatic properties in comparison with the initial GlucDH variant carrying the E170K and the Q252L substitution.

To identify the different properties of the mutated enzymes the following procedure was applied:

- Determination of enzymatic activity
  Measurement under standard conditions with cNAD and Glucose in saturated concentrations for the enzyme
- Determination of Km value for glucose:

  Enzyme activity assay was performed with different glucose concentrations and fixed cNAD concentration.

- Temperature stability:

  Determination of temperature with 50 % remaining activity after 30 minutes incubation:

  Incubation of sample at elevated temperatures for 30 Min to estimate temperature at which GlucDH sample still contains 50 % of its starting activity => higher values = better temperature stability

- Determination of Km value for cNAD:

  Determination was performed as for the determination of Km value for glucose with the difference that the glucose concentration was fixed and cNAD concentration was varied.

- Substrate specificity:

  Measurement with xylose, maltose or galactose instead of glucose as substrate => comparison to reference measurement => statement to substrate specificity => lower values= better specificity to glucose

In detail, samples were prepared as follows:

The clones were cultivated in LB -Amp media (250ml at 37°C, 150 rpm for 19 h), cell disrupted (French press at ca.800 bar), and raw purified (centrifugation of disrupted cells). The obtained supernatants were used as sample.

Activity was determined by adding 50 $\mu$l of sample to 1.5 ml 2.06 mM cNAD;147 mM glucose;167 mM NaCl; 83 mM Tris pH 8.0 in a cuvette (1 cm light path lenght) at 25°C and monitoring the absorbance increase at 360 nm for 5 minutes. The dE/min was calculated

KM value determinations were performed by reducing the glucose concentration at the activity assay (see above) stepwise. The KM value is the substrate concentration at which an enzyme reaction rate is at half-maximum.Temperature with 50 % remaining activity after 30 minutes incubation was assessed as follows:

The GlucDH samples were incubated in 50 mM Tris pH 8.0 , incubated for 30 minutes at 50°C and the activity tested (see above). Afterwards the samples (new samples, i.e. not the ones that were stressed before at 50°C) were incubated at 55°C for 30 minutes and the remaining activity determined as described before. This procedure was continued by increasing the temperature in 5°C steps until no significant activity could be detected. A plot of the remaining activity values in % to the incubation temperature allows detection of the temperature at which 50% activity is still measurable

Determination of Km value for cNAD was performed as for glucose with the difference that cNAD concentration was varied and glucose concentration was fixed at a saturated value.

Substrate specificity was determined as follows:

Activity assay was performed as above described. Only the applied sugars were exchanged. Xylose, maltose and galactose conversion was determined by exchanging the sugar substrate at equimolar amounts.

The value from the reference measurement with glucose as substrate was set to 100% activity. The values obtained with the other sugars were compared to the reference measurement and calculated in percent activity

$$\left(\frac{\frac{dE}{min}\text{ obtained with Xylose (or maltose or galactose)}}{\frac{dE}{min}\text{ obtained with glucose in saturation}}\right) * 100 = \text{activity in percent}$$

The results are summarized in Table 3 below.

TABLE 3

| Mutant | $K_m$-value Glucose [mM] | $K_m$-value cNAD [mM] | Xylose [%] | Maltose [%] | Galactose [%] | Temperature [°C] with 50 % remaining activity after 30 minutes incubation |
|---|---|---|---|---|---|---|
| **GlucDH Mut.A** | 56,0 | 0,80 | 3,81 | 1,48 | 0,09 | 80* |
| **GlucDH Mut.B** | 44,4 | 0,54 | 1,76 | 0,66 | 0,03 | 57,5 |
| **Variant 2** | 39,5 | 0,35 | 0,53 | 0,20 | 0,02 | 76 |
| **Variant 34** | 18,8 | 0,95 | 3,19 | 1,12 | 0,05 | 63,5 |

* no experiment conducted as described above, value estimated from other screening experiments

SEQUENCES

Wild-type GlucDH from *Bacillus subtilis* (SEQ ID NO: 1)

```
MYPDLKGKVV AITGAASGLG KAMAIRFGKE QAKVVINYYS NKQDPNEVKE EVIKAGGEAV     60
VVQGDVTKEE DVKNIVQTAI KEFGTLDIMI NNAGLENPVP SHEMPLKDWD KVIGTNLTGA    120
FLGSREAIKY FVENDIKGNV INMSSVHEVI PWPLFVHYAA SKGGIKLMTE TLALEYAPKG    180
IRVNNIGPGA INTPINAEKF ADPKQKADVE SMIPMGYIGE PEEIAAVAVW LASKESSYVT    240
GITLFADGGM TQYPSFQAGR G                                              261
```

GlucDH Mut.A: Wild-type GlucDH from *Bacillus subtilis* + E170K+Q252L (SEQ ID NO:2)

```
MYPDLKGKVV AITGAASGLG KAMAIRFGKE QAKVVINYYS NKQDPNEVKE EVIKAGGEAV     60
VVQGDVTKEE DVKNIVQTAI KEFGTLDIMI NNAGLENPVP SHEMPLKDWD KVIGTNLTGA    120
FLGSREAIKY FVENDIKGNV INMSSVHEVI PWPLFVHYAA SKGGIKLMTK TLALEYAPKG    180
IRVNNIGPGA INTPINAEKF ADPKQKADVE SMIPMGYIGE PEEIAAVAVW LASKESSYVT    240
GITLFADGGM TLYPSFQAGR G                                              261
```

GlucDH Mut.B: GlucDH Mut.A+G163A+E223W+S237N (SEQ ID NO: 3)

```
MYPDLKGKVV AITGAASGLG KAMAIRFGKE QAKVVINYYS NKQDPNEVKE EVIKAGGEAV     60
VVQGDVTKEE DVKNIVQTAI KEFGTLDIMI NNAGLENPVP SHEMPLKDWD KVIGTNLTGA    120
FLGSREAIKY FVENDIKGNV INMSSVHEVI PWPLFVHYAA SKAGIKLMTK TLALEYAPKG    180
IRVNNIGPGA INTPINAEKF ADPKQKADVE SMIPMGYIGE PEWIAAVAVW LASKESNYVT    240
GITLFADGGM TLYPSFQAGR G                                              261
```

Variant 2: GlucDH Mut.A+L95I+N97S (SEQ ID NO: 4)

```
MYPDLKGKVV AITGAASGLG KAMAIRFGKE QAKVVINYYS NKQDPNEVKE EVIKAGGEAV    60
VVQGDVTKEE DVKNIVQTAI KEFGTLDIMI NNAGIESPVP SHEMPLKDWD KVIGTNLTGA   120
FLGSREAIKY FVENDIKGNV INMSSVHEVI PWPLFVHYAA SKGGIKLMTK TLALEYAPKG   180
IRVNNIGPGA INTPINAEKF ADPKQKADVE SMIPMGYIGE PEEIAAVAVW LASKESSYVT   240
GITLFADGGM TLYPSFQAGR G                                             261
```

Variant 34: GlucDH Mut.B+I80L+L95I+Y39E (SEQ ID NO: 5)

```
MYPDLKGKVV AITGAASGLG KAMAIRFGKE QAKVVINYES NKQDPNEVKE EVIKAGGEAV    60
VVQGDVTKEE DVKNIVQTAL KEFGTLDIMI NNAGIENPVP SHEMPLKDWD KVIGTNLTGA   120
FLGSREAIKY FVENDIKGNV INMSSVHEVI PWPLFVHYAA SKAGIKLMTK TLALEYAPKG   180
IRVNNIGPGA INTPINAEKF ADPKQKADVE SMIPMGYIGE PEWIAAVAVW LASKESNYVT   240
GITLFADGGM TLYPSFQAGR G                                             261
```

## Claims

1. A variant of a Glucose Dehydrogenase (GlucDH) derived from *Bacillus subtilis,* wherein said variant comprises an amino acid sequence having at least 90% identity to SEQ ID NO:1 and wherein said variant comprises a substitution of amino acid residue glutamic acid at a position corresponding to position 170 of SEQ ID NO: 1 with an amino acid residue lysine and a substitution of amino acid residue glutamine at a postion corresponding to position 252 of SEQ ID NO: 1 with an amino acid residue leucine, and wherein said variant further comprises one or more additional amino acid substitutions selected from the group consisting of a substitution of amino acid residue leucine at a position corresponding to position 95 of SEQ ID NO: 1 with an amino acid residue isoleucine or valine, a substitution of amino acid residue asparagine at a position corresponding to position 97 of SEQ ID NO: 1 with an amino acid residue serine, a substitution of amino acid residue glycine at a position corresponding to position 163 of SEQ ID NO: 1 with an amino acid residue alanine, a substitution of amino acid residue glutamic acid at a position corresponding to position 223 of SEQ ID NO: 1 with an amino acid residue phenylalanine, tryptophan, isoleucine, leucine, threonine or tyrosine, and a substitution of amino acid residue serine at a position corresponding to position 237 of

SEQ ID NO: 1 with an amino acid residue glutamic acid, arginine or asparagine.

**2.** The variant according to claim 1, wherein said variant comprises the additional amino acid substitutions of amino acid residue glutamic acid at the position corresponding to position 223 of SEQ ID NO: 1 with an amino acid residue phenylalanine, tryptophan, isoleucine, leucine, threonine or tyrosine, and of amino acid residue serine at the position corresponding to position 237 of SEQ ID NO: 1 with an amino acid residue glutamic acid, arginine or asparagine.

**3.** The variant according to claim 1, wherein said variant comprises the additional amino acid substitutions of amino acid residue glutamic acid at the position corresponding to position 223 of SEQ ID NO: 1 with an amino acid residue phenylalanine, tryptophan, isoleucine, leucine, threonine or tyrosine, of amino acid residue serine at the position corresponding to position 237 of SEQ ID NO: 1 with an amino acid residue glutamic acid, arginine or asparagine and of amino acid residue glycine at the position corresponding to position 163 of SEQ ID NO:1 with an amino acid residue alanine.

**4.** The variant according to claim 3, wherein said variant further comprises one or more additional amino acid substitutions, wherein the amino acid at the position corresponding to

- position 39 of SEQ ID NO: 1 is substituted with Glu (39Glu);
- position 40 of SEQ ID NO: 1 is substituted with Cys (40Cys);
- position 46 of SEQ ID NO: 1 is substituted with Asp (46Asp);
- position 70 of SEQ ID NO: 1 is substituted with Cys (70Cys);
- position 78 of SEQ ID NO: 1 is substituted with Ala (78Ala);
- position 80 of SEQ ID NO: 1 is substituted with Leu (80Leu);
- position 96 of SEQ ID NO: 1 is substituted with Leu (96Leu), Gln (96Gln), Val (96Val), or Met (96Met);
- position 107 of SEQ ID NO: 1 is substituted with Glu (107Glu);
- position 134 of SEQ ID NO: 1 is substituted with Glu (134Glu);
- position 178 of SEQ ID NO: 1 is substituted with Ser (178Ser);
- position 201 of SEQ ID NO: 1 is substituted with Ser (201 Ser);
- position 205 of SEQ ID NO: 1 is substituted with Lys (205Lys); and/or
- position 255 of SEQ ID NO: 1 is substituted with Cys (255Cys).

**5.** The variant according to claim 1, wherein said variant comprises at least one additional amino acid substitution selected from the group consisting of the substitution of amino acid residue leucine at the position corresponding to position 95 of SEQ ID NO: 1 with an amino acid residue isoleucine or valine and the substitution of amino acid residue asparagine at the position corresponding to position 97 of SEQ ID NO: 1 with an amino acid residue serine, said variant optionally further comprising additional amino acid substitutions of amino acid residue tyrosine at a position corresponding to position 39 of SEQ ID NO: 1 with an amino acid residue glutamic acid and/or of amino acid residue serine at a position corresponding to position 40 of SEQ ID NO: 1 with an amino acid residue cysteine.

**6.** The variant according to any of claims 1 to 5, wherein said variant comprises or consists of an amino acid sequence that has at least 95% identity to SEQ ID NO: 1.

**7.** An isolated polynucleotide encoding the GlucDH variant protein according to any of claims 1 to 6.

**8.** An expression vector comprising an isolated polynucleotide as defined in claim 7 operably linked to a promoter sequence capable of promoting the expression of said polynucleotide in a host cell.

**9.** A host cell comprising the expression vector of claim 8.

**10.** A process for producing GlucDH variants comprising culturing the host cell of claim 9 under conditions suitable for production of the enzyme variants.

**11.** A method of detecting, determining or measuring glucose in a sample using a GlucDH variant according to any of claims 1 to 6, comprising contacting the sample with said variant.

**12.** The method of claim 11 further **characterized in that** said detection, determination or measurement of glucose is performed using a sensor or test strip device.

**13.** Use of a GlucDH variant according to any of claims 1 to 6 for determining the amount or concentration of glucose in a sample.

**14.** A device for the detection or measurement of glucose in a sample comprising a GlucDH variant according to any of claims 1 to 6 and other reagents required for said measurement.

**15.** The device according to claim 14, **characterized in that** the device is or comprises a sensor, preferably an electrochemical sensor or an optical sensor, or a test strip, particularly a test strip.

Europäisches Patentamt
European Patent Office
Office européen des brevets

# EUROPEAN SEARCH REPORT

Application Number
EP 16 20 6479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BO LIANG ET AL: "Simultaneously improving stability and specificity of cell surface displayed glucose dehydrogenase mutants to construct whole-cell biocatalyst for glucose biosensor application", BIORESOURCE TECHNOLOGY., vol. 147, 1 November 2013 (2013-11-01), pages 492-498, XP055344979, GB ISSN: 0960-8524, DOI: 10.1016/j.biortech.2013.08.088 * abstract * * page 495; figure 2 * * page 496, right-hand column, last paragraph; figure 3 * ----- | 1,5-15 | INV. C12N9/04 C12N15/52 |
| A | BAIK SANG-HO ET AL: "Cooperative effect of two surface amino acid mutations (Q252L and E170K) in glucose dehydrogenase from Bacillus megaterium IWG3 on stabilization of its oligomeric state", APPLIED AND ENVIRONMENTAL MICROBIOLOGY, AMERICAN SOCIETY FOR MICROBIOLOGY, US, vol. 71, no. 6, 1 June 2005 (2005-06-01), pages 3285-3293, XP002580338, ISSN: 0099-2240, DOI: 10.1128/AEM.71.6.3285-3293.2005 * abstract * * page 3286; table 1 * ----- | 1,5-15 | TECHNICAL FIELDS SEARCHED (IPC) C12N |

-/--

2

~~The present search report has been drawn up for all claims~~

| Place of search | Date of completion of the search | Examiner |
|---|---|---|
| Munich | 14 February 2017 | Niebuhr-Ebel, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 16 20 6479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | S.-H. BAIK ET AL: "Significantly enhanced stability of glucose dehydrogenase by directed evolution", APPLIED MICROBIOLOGY AND BIOTECHNOLOGY, vol. 61, no. 4, 5 March 2003 (2003-03-05), pages 329-335, XP055344908, DE ISSN: 0175-7598, DOI: 10.1007/s00253-002-1215-1 * abstract * * page 332, right-hand column, last paragraph - page 334, left-hand column, paragraph f; figures 1,2 * ----- | 1,5-15 | |
| A | WO 2011/020856 A1 (HOFFMANN LA ROCHE [CH]; ROCHE DIAGNOSTICS GMBH [DE]; ROEDEL WOLFGANG [) 24 February 2011 (2011-02-24) * page 7, line 3 - line 12 * * page 19, line 20 - line 24; figure 8 * ----- | 1,5-15 | |
| A | WO 2005/045016 A2 (CODEXIS INC [US]) 19 May 2005 (2005-05-19) * page 29, paragraph 89 * * table 1 * * page 32 - page 33; table 2 * ----- | 1,5-15 | TECHNICAL FIELDS SEARCHED (IPC) |

~~The present search report has been drawn up for all claims~~

2

| Place of search | Date of completion of the search | Examiner |
|---|---|---|
| Munich | 14 February 2017 | Niebuhr-Ebel, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 16 20 6479

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1, 5-15(all partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 16 20 6479

The Search Division considers that the present European patent application does not comply with the requirements of unity of invention and relates to several inventions or groups of inventions, namely:

1. claims: 1, 5-15(all partially)

   Variant of a Glucose Dehydrogenase (GlucDH) derived from Bacillus subtilis, wherein said variant comprises an amino acid sequence having at least 90% identity to SEQ ID NO:1 and wherein said variant comprises a substitution E170K and a substitution Q252L, and wherein said variant further comprises a substitution L95I or L95V

---

2. claims: 1, 5-15(all partially)

   Variant of a Glucose Dehydrogenase (GlucDH) derived from Bacillus subtilis, wherein said variant comprises an amino acid sequence having at least 90% identity to SEQ ID NO:1 and wherein said variant comprises a substitution E170K and a substitution Q252L, and wherein said variant further comprises a substitution N97S

---

3. claims: 1, 5-15(all partially)

   Variant of a Glucose Dehydrogenase (GlucDH) derived from Bacillus subtilis, wherein said variant comprises an amino acid sequence having at least 90% identity to SEQ ID NO:1 and wherein said variant comprises a substitution E170K and a substitution Q252L, and wherein said variant further comprises a substitution G163A

---

4. claims: 1-15(partially)

   Variant of a Glucose Dehydrogenase (GlucDH) derived from Bacillus subtilis, wherein said variant comprises an amino acid sequence having at least 90% identity to SEQ ID NO:1 and wherein said variant comprises a substitution E170K and a substitution Q252L, and wherein said variant further comprises a substitution E223(F,W,I,L,T or Y)

---

5. claims: 1-15(partially)

   Variant of a Glucose Dehydrogenase (GlucDH) derived from Bacillus subtilis, wherein said variant comprises an amino acid sequence having at least 90% identity to SEQ ID NO:1 and wherein said variant comprises a substitution E170K and a substitution Q252L, and wherein said variant further comprises a substitution E237(N, R or E)

---

# ANNEX TO THE EUROPEAN SEARCH REPORT ON EUROPEAN PATENT APPLICATION NO. EP 16 20 6479

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2017

| Patent document cited in search report | Publication date | Patent family member(s) | Publication date |
|---|---|---|---|
| WO 2011020856 A1 | 24-02-2011 | CA 2770249 A1 | 24-02-2011 |
| | | CN 102471767 A | 23-05-2012 |
| | | EP 2292751 A1 | 09-03-2011 |
| | | EP 2467478 A1 | 27-06-2012 |
| | | JP 5851401 B2 | 03-02-2016 |
| | | JP 2013502212 A | 24-01-2013 |
| | | KR 20120064076 A | 18-06-2012 |
| | | KR 20150070443 A | 24-06-2015 |
| | | US 2012276565 A1 | 01-11-2012 |
| | | US 2017073661 A1 | 16-03-2017 |
| | | WO 2011020856 A1 | 24-02-2011 |
| WO 2005045016 A2 | 19-05-2005 | AU 2004288134 A1 | 19-05-2005 |
| | | BR PI0413492 A | 17-10-2006 |
| | | CA 2535147 A1 | 19-05-2005 |
| | | EP 1660648 A2 | 31-05-2006 |
| | | JP 2007502114 A | 08-02-2007 |
| | | KR 20060064620 A | 13-06-2006 |
| | | MX PA06001719 A | 19-05-2006 |
| | | US 2005095619 A1 | 05-05-2005 |
| | | US 2010304459 A1 | 02-12-2010 |
| | | WO 2005045016 A2 | 19-05-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0354441 A2
- EP 0431456 A1
- EP 0302287 A2
- EP 1593434 A2
- WO 200701249 A
- WO 2011012270 A
- WO 2009103540 A
- US 20050214891 A
- WO 2007012494 A
- WO 2014195363 A
- US 6541216 B
- EP 262445 A
- US 4816224 A
- US 5413690 A
- US 5762770 A
- US 5997817 A

### Non-patent literature cited in the description


- **HONES.** *Diabetes Technology & Therapeutics,* 2008, vol. 10, S10
- **N. J. OPPENHEIMER.** The Pyridine Nucleotide Coenzymes. Academic Press, 1982, 56-65
- **HILT et al.** *Biochim Biophys Acta.,* 29 January 1991, vol. 1076 (2), 298-304
- **S.-H. BAIK et al.** *Appl. Microbial Biotechnol.,* 2003, vol. 61, 329-335
- **SAMBROOK et al.** Molecular Cloning: A laboratory manual. 2000
- **BENJAMIN LEWIN.** Genes V. Oxford University Press, 1994
- The Encyclopedia of Molecular Biology. Blackwell Science Ltd, 1994
- Molecular Biology and Biotechnology: a Comprehensive Desk Reference. VCH Publishers, Inc, 1995
- **AUSUBEL, F. et al.** Current protocols in molecular biology. Wiley Verlag, 1994